(19) Europäisches Patentamt
European Patent Office
Office européen des brevets

(11) Publication number: **0 241 254**
**A2**

(12) # EUROPEAN PATENT APPLICATION

(21) Application number: 87302988.8

(22) Date of filing: 06.04.87

(51) Int. Cl.³: C 07 D 405/12
C 07 D 409/12, A 01 N 47/36
//C07D333/62

(30) Priority: 07.04.86 US 849252
23.10.86 US 922261

(43) Date of publication of application:
14.10.87 Bulletin 87/42

(84) Designated Contracting States:
AT BE CH DE ES FR GB GR IT LI LU NL SE

(71) Applicant: E.I. DU PONT DE NEMOURS AND COMPANY
1007 Market Street
Wilmington Delaware 19898(US)

(72) Inventor: Amuti, Kofi Sam
5412 Valley Green Drive
Wilmington Delaware 19808(US)

(72) Inventor: Wolf, Anthony David
151 Kirkcaldy Drive
Elkton Maryland 21921(US)

(74) Representative: Hildyard, Edward Martin et al,
Frank B. Dehn & Co. Imperial House 15-19 Kingsway
London WC2B 6UZ(GB)

(54) **Herbicidal sulfonamides.**

(57) Compounds of the formula:

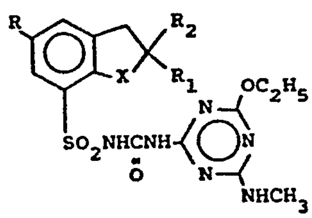

wherein
R is H, Cl, F, $CH_3$ or $OCH_3$;
$R_1$ is H or $CH_3$;
$R_2$ is H, $CH_3$ or $C_2H_5$; and
X is O, S or $SO_2$;
provided that when $R_1$ and $R_2$ are simultaneously H, then X is O, and agriculturally suitable salts thereof exhibit herbicidal activity and in particular show selectivity as herbicides for broad spectrum weed control in oilseed rape.

The compounds may be made by a variety of synthetic routes, e.g. by reacting the appropriate benzothiophenesulfonylisocyanate with the appropriate aminotriazine.

EP 0 241 254 A2

Croydon Printing Company Ltd.

"HERBICIDAL SULFONAMIDES"

This invention relates to N-heterocyclicaminocarbonyl-benzothiophenesulfonamides which are useful as agricultural chemicals and in particular show selectivity as herbicides for broad spectrum weed control in oilseed rape.

U.S. 4,514,211 discloses herbicidal sulfonamides of formula

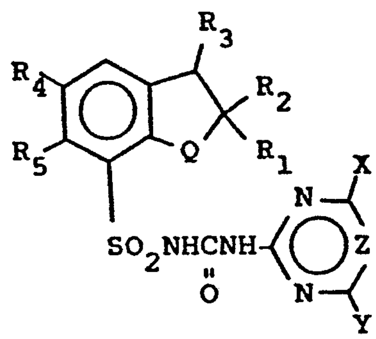

wherein

Q is O, S, SO or $SO_2$;

$R_1$ is H or $C_1$-$C_4$ alkyl;

$R_2$ is H or $C_1$-$C_4$ alkyl;

$R_3$ is H or $CH_3$;

$R_4$ is H, Cl, $CH_3$, $CF_3$, $OCH_3$, Br, F, $SCH_3$ or $OCF_2H$;

$R_5$ is H, $CH_3$, $OCH_3$, Cl, Br, $NO_2$, $CO_2R_7$, $SO_2R_8$, $OSO_2R_9$, $SO_2NR_{10}R_{11}$, F, $CF_3$, $SCH_3$, $OCF_2H$ or $SO_2N(OCH_3)CH_3$;

X is H, $CH_3$, $OCH_3$, Cl, F, $OCF_2H$ or $SCF_2H$;

Y is $CH_3$, $OCH_3$, $OC_2H_5$, $CH_2OCH_3$, $NH_2$, $NHCH_3$, $N(CH_3)_2$, $CH(OCH_3)_2$, $CH(OCH_2CH_3)_2$, $C_2H_5$, $CF_3$, $CH_2$=$CHCH_2O$, $CF_3CH_2O$, $OCH_2CH_2Cl$,

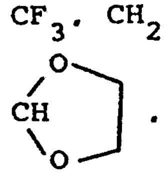 , 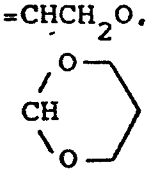 , 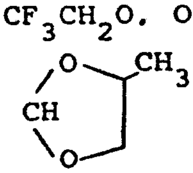 , HC≡$CCH_2O$,

$OCH_2CH_2Br$, $OCH_2CH_2F$, CN, $CH_2OCH_2CH_3$, $OCH_2CH_2OCH_3$ or $GCF_2T$ wherein G is O or S and T is H, CHClF, CHBrF, $CF_2H$ or $CHFCF_3$.

U.S. 4,492,596 discloses herbicidal sulfonylureas of formula

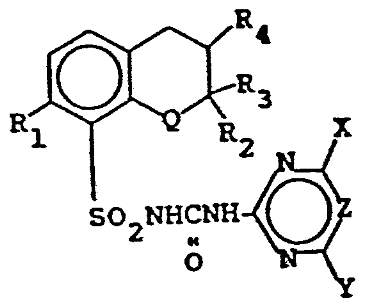

wherein

Q is O, S or $SO_2$;

$R_1$ is H, $CH_3$, $OCH_3$, Cl, Br, $CO_2R_5$, $SO_2R_6$, $OSO_2R_7$ or $SO_2NR_8R_9$;

$R_4$ is H or $CH_3$;

$R_2$ and $R_3$ are independently H or $C_1$-$C_3$ alkyl;

X is $CH_3$, $OCH_3$ or Cl;

Y is $CH_3$, $OCH_3$, $OC_2H_5$, $CH_2OCH_3$, $C_2H_5$, $NH_2$, $NHCH_3$ or $N(CH_3)_2$; and

Z is CH or N.

Compounds of Formula I below are also generically disclosed in EP-A-99,339 which discloses herbicidal sulfonamides of formula

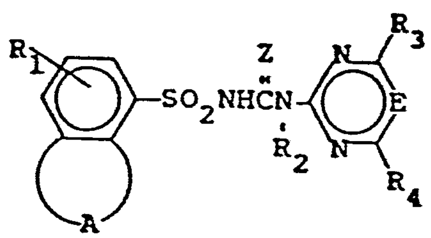

wherein

A is an unsubstituted or substituted bridge of 3 or 4 atoms which contains 1 or 2 oxygen, sulfur or nitrogen atoms and, together with the linking carbon atom, forms a nonaromatic 5- or 6-membered heterocyclic ring

system, with the proviso that two oxygen atoms are separated by at least one carbon atom and that oxygen and sulfur atoms are only linked to each other if the sulfur atom takes the form of the -SO- or $SO_2$- group, and $R_2$ is hydrogen, halogen, nitro, $C_1$-$C_4$ alkyl, $C_1$-$C_4$ haloalkyl, $C_1$-$C_4$ alkoxy, $C_1$-$C_2$ haloalkoxy, $C_1$-$C_4$ alkoxycarbonyl, $C_1$-$C_4$ alkylthio, $C_1$-$C_4$ alkylsulfinyl, $C_1$-$C_4$ alkylsulfonyl or $C_2$-$C_5$ alkoxyalkoxy;

$R_1$ is H, halogen, $NO_2$, $C_1$-$C_4$ alkyl, $C_1$-$C_4$ haloalkyl, $C_1$-$C_4$ alkoxy, $C_1$-$C_4$ haloalkoxy, $C_1$-$C_4$ alkoxycarbonyl, $C_1$-$C_4$ alkylthio, $C_1$-$C_4$ alkylsulfinyl, $C_1$-$C_4$ alkylsulfonyl or $C_2$-$C_5$ alkoxyalkoxy;

$R_3$ and $R_4$, each independently of the other, are hydrogen, $C_1$-$C_4$ alkyl, $C_1$-$C_4$ alkoxy, $C_1$-$C_4$ haloalkoxy, $C_1$-$C_4$ haloalkylthio, $C_1$-$C_4$ alkylthio, halogen, $C_2$-$C_5$ alkoxyalkoxy or $NR_5R_6$, wherein $R_5$ and $R_6$ are hydrogen or $C_1$-$C_4$ alkyl; and

E is CH or N.

U.S. 4,548,638 discloses herbicidal sulfonamides of formula

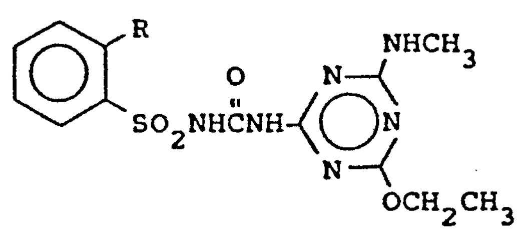

wherein

R is $CO_2CH_3$, $CO_2CH_2CH_3$, $CO_2CH_2CH_2CH_3$, $CO_2CH_2CH{=}CH_2$, $CO_2CH(CH_3)_2$, $CO_2CH_2CH_2Cl$, $SO_2N(CH_3)_2$ or $OSO_2CH_3$, which are useful for the control of undesirable vegetation in rape.

## SUMMARY OF THE INVENTION

This invention relates to novel compounds of Formula I, agriculturally suitable compositions containing them, and their method-of-use as pre-emergent or postemergent herbicides or as plant growth regulants. The compounds are especially useful as selective herbicides for oilseed rape and cultivated mustards.

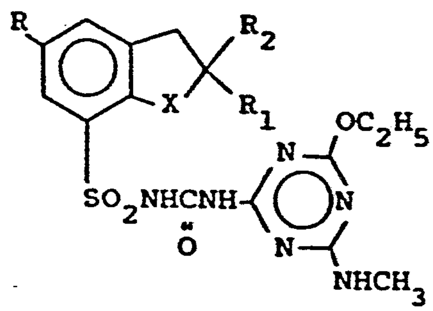

I

wherein

R is H, Cl, F, $CH_3$ or $OCH_3$;

$R_1$ is H or $CH_3$;

$R_2$ is H, $CH_3$ or $C_2H_5$; and

X is O, S or $SO_2$;

provided that when $R_1$ and $R_2$ are simultaneously H, then X is O.

Preferred for reasons of increased ease of synthesis and/or greater herbicidal efficacy are:

1) Compounds of Formula I where R is H.

2) Compounds of Preferred 1 where $R_1$ is H; and $R_2$ is $CH_3$;

Specifically Preferred for reasons of its most favorable ease of synthesis and/or highest herbicidal activity is:

- N-[[4-ethoxy-6-(methylamino)-1,3,5-triazin-2-yl]aminocarbonyl]-2,3-dihydro-2-methylbenzo-[b]thiophene-7-sulfonamide, 1,1-dioxide, m.p. 143-150°C(d).

## DETAILED DESCRIPTION OF THE INVENTION

The compounds of Formula I can be prepared by one or more of the methods described below in Equations 1, 2 and 3. Reaction conditions are given by way of illustration.

As shown in Equation 1, compounds of Formula I can be prepared by reacting a sulfonylisocyanate of Formula II with the heterocyclic amine of Formula III, where $R_1$ = H or $CH_3$, R = H, F, Cl, $CH_3$ or $OCH_3$, and $R_2$ = H, $CH_3$ or $C_2H_5$.

Equation 1

$$\underset{\text{II}}{JSO_2NCO} + \underset{\text{III}}{NH_2\text{-}A} \longrightarrow \underset{\text{I}}{JSO_2NH\overset{O}{\overset{\|}{C}}NHA}$$

where J is 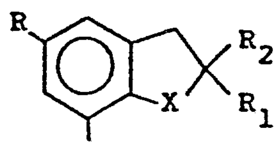 ; and

A is 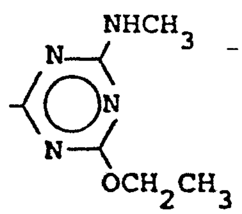

The reaction is carried out at 25° to 100°C in an inert, aprotic solvent such as methylene chloride or acetonitrile for 0.5 to 24 hours as taught in U.S. Patent 4,127,405.

Alternatively, compounds of Formula I can be prepared by reacting a sulfonylcarbamate of Formula IV with the amine of Formula III, as shown in Equation 2.

Equation 2

$JSO_2NHCO_2Ph$ + III → I

IV

where J is as defined in Equation 1.

The reaction is carried out at 50° to 100°C in a solvent such as dioxane for 0.5 to 24 hours as taught in EPO Publication No. 44,807. The required carbamates IV are prepared by reacting the corresponding sulfonamides V with diphenylcarbonate in the presence of a strong base.

Alternatively, compounds of Formula I may be prepared as shown in Equation 3, by the reaction of a sulfonamide V with the appropriate carbamate heterocycle, VI.

Equation 3

$JSO_2NH_2$ + $C_6H_5O\overset{O}{\overset{\parallel}{C}}NH\text{-}A$ → I

V VI

where A and J are as defined in Equation 1.

The reaction of Equation 3 is best carried out according to the methods taught in EPO Publication No. 70,804.

The intermediate sulfonylisocyanates of Formula II can be prepared by the two-step procedure shown below in Equation 4, starting from the appropriate sulfonamides, V.

Equation 4

a) V + $SOCl_2$ → $JSO_2NSO$

VII

b) VII + $COCl_2$ ⟶ II

where J is as defined in Equation 1.

The reactions of Equation 4a and 4b are best carried out according to the procedure of Ulrich, et al. as described in J. Org. Chem., 34, 3200 (1969). The sulfonamide V is boiled under reflux with an excess of thionyl chloride which functions as both a reactant and solvent. When the sulfonamide protons are no longer detectable by proton NMR (15-20 hrs. on the average), the thionyl chloride is removed under reduced pressure and the residue is dissolved in an inert solvent such as toluene, benzene, xylenes, etc. A catalytic amount of pyridine is added. The mixture is treated with at least one equivalent of phosgene and heated to 60°-140°C with 80°-100°C preferred. Conversion to the isocyanate is substantially complete within about 1/4 to 3 hours. The mixture containing the sulfonyl isocyanate can be used directly or the sulfonyl isocyanate can be isolated in pure form by filtration and evaporation of the filtrate followed by vacuum distillation if necessary.

The sulfonyl isocyanates II can also be prepared from the sulfonamides V by a two step procedure involving (a) reacting the sulfonamides with n-butyl isocyanate in the presence of a base such as $K_2CO_3$ at reflux in an inert solvent such as 2-butanone or acetonitrile, forming a n-butyl sulfonylurea; and (b) reacting this compound with phosgene and a tertiary amine catalyst at reflux in xylene solvent. This method is similar to a procedure taught by Ulrich and Sayigh, Newer Methods of Preparative Organic Chemistry, Vol. VI, p. 223-241, Academic Press, New York and London, W. Forest Ed.

The sulfonamides of Formula V may be prepared by the methods described in U.S. 4,514,211.

The aminotriazine III is known in the art. For example, see J. Amer. Chem. Soc., 71, 3248 (1949).

Agriculturally suitable salts of compounds of Formula I are also useful herbicides and can be prepared in a number of ways known to the art. For example, metal salts can be made by contacting compounds of Formula I with a solution of an alkali or alkaline earth metal salt having a sufficiently basic anion (e.g., hydroxide, alkoxide, carbonate or hydroxide). Quaternary amine salts can be made by similar techniques.

Salts of compounds of Formula I can also be prepared by exchange of one cation for another. Cationic exchange can be effected by direct contact of an aqueous solution of a salt of a compound of Formula I (e.g., alkali or quaternary amine salt) with a solution containing the cation to be exchanged. This method is most effective when the desired salt containing the exchanged cation is insoluble in water and can be separated by filtration.

Exchange may also be effected by passing an aqueous solution of a salt of a compound of Formula I (e.g., an alkali metal or quaternary amine salt) through a column packed with a cation exchange resin containing the cation to be exchanged for that of the original salt and the desired product is eluted from the column. This method is particularly useful when the desired salt is water-soluble, e.g., a potassium sodium or calcium salt.

Acid addition salts, useful in this invention, can be obtained by reacting a compound of Formula I with a suitable acid, e.g., p-toluenesulfonic acid, trichloroacetic acid or the like.

The preparation of the compounds of this invention is further illustrated by the following specific examples. Unless otherwise indicated, temperatures are in degrees centigrade.

EXAMPLE 1

2,3-dihydro-2-methyl benzo[b]thiophene-7-sulfonyl isocyanate, 1,1-dioxide

A suspension of 2.6 g of 2,3-dihydro-2-methylbenzo[b]thiophene-7-sulfonamide, 1,1-dioxide in 25 mL of thionyl chloride was refluxed under nitrogen for 48 hrs. The resultant solution was allowed to cool and excess thionyl chloride was removed *in vacuo*. The residue was dissolved in 50 mL of xylenes and again concentrated. To this residue was added 2 drops of pyridine and 50 mL of xylenes. The mixture was heated to reflux and 1.5 mL of condensed phosgene was added dropwise with temperature being maintained at greater than 130°C. After the addition was complete the mixture was refluxed at 130-140°C for three hours, then allowed to cool and the volatile materials removed *in vacuo*. The residual brown material was dissolved in methylene chloride and used without further purification.

IR($CH_2Cl_2$) 2242 $cm^{-1}$ ($SO_2NCO$).

EXAMPLE 2

N-[[4-ethoxy-6-(methylamino)-1,3,5-triazin-2-yl)aminocarbonyl]-2,3-dihydro-2-methyl benzo[b]thiophene-7-sulfonamide, 1,1-dioxide

To a 10 mL aliquot of the product from Example 1 containing 1.4 g of the isocyanate was added 0.82 g of 2-amino-4-ethoxy-6-(methylamino)-1,3,5-triazine and the resultant suspension was stirred at room temperature overnight. The small amount of undissolved

solids were collected by filtration and discarded. The filtrate was concentrated in vacuo and triturated with n-butylchloride. The resultant solid was collected by filtration then slurried in 10 mL of 5% of HCl for 10 min. The remaining white solid was collected by filtration, washed with distilled water and dried to provide 0.65 g of the title compound. m.p. 143-150°C(d) IR. (Nujol) 1712 $cm^{-1}$ (C=O).

NMR($CDCl_3$) δ: 1.38 (3H, t);
1.65 (3H, d);
2.94 (4H, m);
3.5 (2H, m);
4.5 (2H, m);
7.5-8.0 (2H, m);
8.26 (1H, m);
7.5-8.2 (1H, br s, NH);
10.2 (1H, s, NH);
13.2 (1H, s, NH).

Using the methods described herein and the procedures of Examples 1 and 2, the compounds of Table I may be prepared by one skilled in the art.

TABLE I

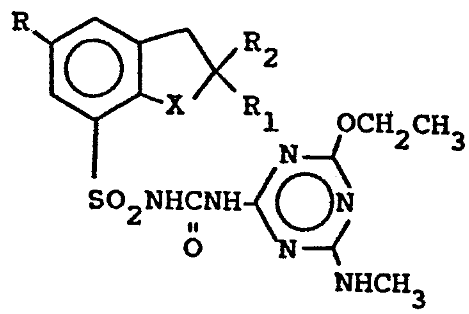

| X | R | $R_1$ | $R_2$ | m.p. (°C) |
|---|---|---|---|---|
| O | H | H | H | 210-214 |
| O | H | H | $CH_3$ | 188-190(d) |
| O | H | H | $C_2H_5$ | |
| O | H | $CH_3$ | $CH_3$ | |
| O | H | $CH_3$ | $C_2H_5$ | |
| O | Cl | H | H | |
| O | Cl | H | $CH_3$ | |
| O | Cl | H | $C_2H_5$ | |
| O | Cl | $CH_3$ | $CH_3$ | |
| O | Cl | $CH_3$ | $C_2H_5$ | |
| O | F | H | H | |
| O | F | H | $CH_3$ | |
| O | F | H | $C_2H_5$ | |
| O | F | $CH_3$ | $CH_3$ | |
| O | F | $CH_3$ | $C_2H_5$ | |
| O | $OCH_3$ | H | H | |
| O | $OCH_3$ | H | $CH_3$ | |
| O | $OCH_3$ | H | $C_2H_5$ | |
| O | $OCH_3$ | $CH_3$ | $CH_3$ | |
| O | $OCH_3$ | $CH_3$ | $C_2H_5$ | |
| O | $CH_3$ | H | H | |
| O | $CH_3$ | H | $CH_3$ | |
| O | $CH_3$ | $CH_3$ | $CH_3$ | |
| O | $CH_3$ | H | $C_2H_5$ | |

TABLE I (continued)

| X | R | $R_1$ | $R_2$ | m.p. (°C) |
|---|---|---|---|---|
| O | $CH_3$ | $CH_3$ | $C_2H_5$ | |
| S | H | H | $CH_3$ | 152-155(d) |
| S | H | H | $C_2H_5$ | |
| S | H | $CH_3$ | $CH_3$ | |
| S | H | $CH_3$ | $C_2H_5$ | |
| S | Cl | H | $CH_3$ | |
| S | Cl | H | $C_2H_5$ | |
| S | Cl | $CH_3$ | $CH_3$ | |
| S | Cl | $CH_3$ | $C_2H_5$ | |
| S | F | H | $CH_3$ | |
| S | F | H | $C_2H_5$ | |
| S | F | $CH_3$ | $CH_3$ | |
| S | F | $CH_3$ | $C_2H_5$ | |
| S | $OCH_3$ | H | $CH_3$ | |
| S | $OCH_3$ | H | $C_2H_5$ | |
| S | $OCH_3$ | $CH_3$ | $CH_3$ | |
| S | $OCH_3$ | $CH_3$ | $C_2H_5$ | |
| S | $CH_3$ | H | $CH_3$ | |
| S | $CH_3$ | H | $C_2H_5$ | |
| S | $CH_3$ | $CH_3$ | $CH_3$ | |
| S | $CH_3$ | $CH_3$ | $C_2H_5$ | |
| $SO_2$ | H | H | $CH_3$ | 143-150(d) |
| $SO_2$ | H | H | $C_2H_5$ | |
| $SO_2$ | H | $CH_3$ | $CH_3$ | |
| $SO_2$ | H | $CH_3$ | $C_2H_5$ | |
| $SO_2$ | Cl | H | $CH_3$ | |
| $SO_2$ | Cl | H | $C_2H_5$ | |
| $SO_2$ | Cl | $CH_3$ | $CH_3$ | |
| $SO_2$ | Cl | $CH_3$ | $C_2H_5$ | |
| $SO_2$ | F | H | $CH_3$ | |
| $SO_2$ | F | H | $C_2H_5$ | |
| $SO_2$ | F | $CH_3$ | $CH_3$ | |
| $SO_2$ | F | $CH_3$ | $C_2H_5$ | |

TABLE I (continued)

| X | R | $R_1$ | $R_2$ | m.p. (°C) |
|---|---|---|---|---|
| $SO_2$ | $OCH_3$ | H | $CH_3$ | |
| $SO_2$ | $OCH_3$ | H | $C_2H_5$ | |
| $SO_2$ | $OCH_3$ | $CH_3$ | $CH_3$ | |
| $SO_2$ | $OCH_3$ | $CH_3$ | $C_2H_5$ | |
| $SO_2$ | $CH_3$ | H | $CH_3$ | |
| $SO_2$ | $CH_3$ | H | $C_2H_5$ | |
| $SO_2$ | $CH_3$ | $CH_3$ | $CH_3$ | |
| $SO_2$ | $CH_3$ | $CH_3$ | $C_2H_5$ | |

Formulations

Useful formulations of the compounds of Formula I can be prepared in conventional ways. They include dusts, granules, pellets, solutions, suspensions, emulsions, wettable powders, emulsifiable concentrates and the like. Many of these may be applied directly. Sprayable formulations can be extended in suitable media and used at spray volumes of from a few liters to several hundred liters per hectare. High strength compositions are primarily used as intermediates for further formulation. The formulations, broadly, contain about 0.1% to 99% by weight of active ingredient(s) and at least one of (a) about 0.1% to 20% surfactant(s) and (b) about 1% to 99.9% solid or liquid inert diluent(s). More specifically, they will contain these ingredients in the following approximate proportions:

Table II

| | Weight Percent* | | |
|---|---|---|---|
| | Active Ingredient | Diluent(s) | Surfactant(s) |
| Wettable Powders | 20-90 | 0-74 | 1-10 |
| Oil Suspensions, Emulsions, Solutions, (including Emulsifiable Concentrates) | 3-50 | 40-95 | 0-15 |
| Aqueous Suspension | 10-50 | 40-84 | 1-20 |
| Dusts | 1-25 | 70-99 | 0-5 |
| Granules and Pellets | 0.1-95 | 5-99.9 | 0-15 |
| High Strength Compositions | 90-99 | 0-10 | 0-2 |

* Active ingredient plus at least one of a Surfactant or a Diluent equals 100 weight percent.

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide," 2nd Ed., Interscience, New York, 1950. Solubility under 0.1% is preferred for suspension concentrates; solution concentrates are preferably stable against phase separation at 0°C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", Chemical Engineering,

December 4, 1967, pp. 147ff. and "Perry's Chemical Engineer's Handbook", 5th Ed., McGraw-Hill, New York, 1973, pp. 8-57ff.

For further information regarding the art of formulation, see for example:

H. M. Loux, U.S. Patent 3,235,361, February 15, 1966, Col. 6, line 16 through Col. 7, line 19 and Examples 10 through 41;

R. W. Luckenbaugh, U.S. Patent 3,309,192, March 14, 1967, Col. 5, line 43 through Col. 7, line 62 and Examples 8, 12, 15, 39, 41, 52, 53, 58, 132, 138-140, 162-164, 166, 167 and 169-182;

H. Gysin and E. Knusli, U.S. Patent 2,891,855, June 23, 1959, Col. 3, line 66 through Col. 5, line 17 and Examples 1-4;

G. C. Klingman, "Weed Control as a Science", John Wiley and Sons, Inc., New York, 1961, pp. 81-96; and

J. D. Fryer and S. A. Evans, "Weed Control Handbook", 5th Ed., Blackwell Scientific Publications, Oxford, 1968, pp. 101-103.

In the following examples, all parts are by weight unless otherwise indicated.

Example 3

Wettable Powder

| | |
|---|---|
| N-[[4-ethoxy-6-(methylamino)-1,3,5-triazin-2-yl]aminocarbonyl]-2,3-dihydro-2-methylbenzo-[b]thiophene-7-sulfonamide, 1,1-dioxide | 80% |
| sodium alkylnaphthalenesulfonate | 2% |
| sodium ligninsulfonate | 2% |
| synthetic amorphous silica | 3% |
| kaolinite | 13% |

The ingredients are blended and ground in a hammermill to produce particles with an average particle size of less than 25 microns in diameter. The material is reblended and sifted through a U.S.S. No. 50 sieve (0.33 mm opening) before being packaged.

Example 4

Wettable Powder

| | |
|---|---|
| N-[[4-ethoxy-6-(methylamino)-1,3,5-triazin-2-yl]aminocarbonyl]-2,3-dihydro-2-methylbenzo-[b]thiophene-7-sulfonamide, 1,1-dioxide | 65% |
| dodecylphenol polyethyelene glycol ether | 2% |
| sodium ligninsulfonate | 4% |
| sodium silicoaluminate | 6% |
| montmorillonite (calcined) | 23% |

The ingredients are thoroughly blended. The liquid surfactant is added by spraying upon the solid ingredients in the blender. After grinding in a hammermill to produce particles essentially all below 100 microns, the material is reblended and sifted through a U.S.S. No. 50 sieve (0.3 mm opening) and packaged.

Example 5

Granule

| | |
|---|---|
| Wettable Powder of Example 4 | 15% |
| gypsum | 69% |
| potassium sulfate | 16% |

The ingredients are blended in a rotating mixer and water sprayed on to accomplish granulation. When most of the material has reached the desired range of 1.0 to 0.42 mm. (U.S.S. No. 18 to 40 sieves), the granules are removed, dried, and screened. Oversize material is crushed to produce additional material in the desired range. These granules contain 12% active ingredient.

Example 6

Extruded Pellet

| | |
|---|---|
| N-[[4-ethoxy-6-(methylamino)-1,3,5-triazin-2-yl]aminocarbonyl]-2,3-dihydro-2-methylbenzo-[b]thiophene-7-sulfonamide, 1,1-dioxide | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammermilled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled.

Example 7

Wettable Powder

| | |
|---|---|
| N-[[4-ethoxy-6-(methylamino)-1,3,5-triazin-2-yl]aminocarbonyl]-2,3-dihydro-2-methylbenzo-[b]thiophene-7-sulfonamide, 1,1-dioxide | 40% |
| dioctyl sodium sulfosuccinate | 1.5% |
| sodium ligninsulfonate | 3% |
| low viscosity methyl cellulose | 1.5% |
| attapulgite | 54% |

The ingredients are thoroughly blended, passed through an air mill, to produce an average particle size under 15 microns, reblended and sifted through a U.S.S. No. 50 sieve (0.3 mm opening) before packaging.

Example 8.

Oil Suspension

| | |
|---|---|
| N-[[4-ethoxy-6-(methylamino)-1,3,5-triazin-2-yl]aminocarbonyl]-2,3-dihydro-2-methylbenzo-[b]thiophene-7-sulfonamide, 1,1-dioxide | 35% |
| blend of polyalcohol carboxylic esters and oil soluble petroleum sulfonates | 6% |
| xylene | 59% |

The ingredients are combined and ground together in a sand mill to produce particles essentially all below 3 microns. The product can be used directly, extended with oils, or emulsified in water.

Example 9

Wettable Powder

| | |
|---|---|
| N-[[4-ethoxy-6-(methylamino)-1,3,5-triazin-2-yl]aminocarbonyl]-2,3-dihydro-2-methylbenzo-[b]thiophene-7-sulfonamide, 1,1-dioxide | 50% |
| sodium alkylnaphthalenesulfonate | 2% |
| low viscosity methyl cellulose | 2% |
| diatomaceous earth | 46% |

The ingredients are blended, coarsely hammermilled and then air milled to produce particles of active essentially all below 10 microns in diameter. The product is reblended before packaging.

Example 10

High Strength Concentrate

| | |
|---|---|
| N-[[4-ethoxy-6-(methylamino)-1,3,5-triazin-2-yl]aminocarbonyl]-2,3-dihydro-2-methylbenzo-[b]thiophene-7-sulfonamide, 1,1-dioxide | 98.5% |
| silica aerogel | 0.5% |
| synthetic amorphous silica | 1.0% |

The ingredients are blended and ground in a hammermill to produce a high strength concentrate essentially all passing a U.S.S. No. 50 screen (0.3 mm openings). This material may then be formulated in a variety of ways.

Example 11

Oil Suspension

| | |
|---|---|
| N-[[4-ethoxy-6-(methylamino)-1,3,5-triazin-2-yl]aminocarbonyl]-2,3-dihydro-2-methylbenzo-[b]thiophene-7-sulfonamide, 1,1-dioxide | 25% |
| polyoxyethylene sorbitol hexaoleate | 5% |
| highly aliphatic hydrocarbon oil | 70% |

The ingredients are ground together in a sand mill until the solid particles have been reduced to under about 5 microns. The resulting thick suspension may be applied directly, but preferably after being extended with oils or emulsified in water.

Example 12

Aqueous Suspension

| | |
|---|---|
| N-[[4-ethoxy-6-(methylamino)-1,3,5-triazin-2-yl]aminocarbonyl]-2,3-dihydro-2-methylbenzo-[b]thiophene-7-sulfonamide, 1,1-dioxide | 25% |
| hydrated attapulgite | 3% |
| crude calcium ligninsulfonate | 10% |
| sodium dihydrogen phosphate | 0.5% |
| water | 61.5% |

The ingredients are ground together in a ball or roller mill until the solid particles have been reduced to diameters under 10 microns.

Utility

The compounds of the present invention are selective herbicides for grass, broadleaf weeds, volunteer cereals control in oilseed rape (Brassica napus and B. campestris), cultivated mustards (Green, Brown, Oriental and Yellow) and cole crops (cabbage, cauliflower, kale, collards and kohlrabi). Control of grass weeds, volunteer cereals and broadleaf weeds can be expected at rates between 8 and 250 g/ha. The compounds can be applied as preemergence or postemergence

treatments and exhibit excellent safety to rape, cultivated mustards, cotton, alfalfa, clovers, cole crops and turnips.

The compounds of this invention may be used in combination with other herbicides. They are particularly useful in combination with broadleaf herbicides used in rape including:

| Common Name or Code Number | Chemical Name |
|---|---|
| | 2-[[(4-ethoxy-6-methylamino-1,3,5-triazin-2-yl)amino carbonyl-]aminosulfonyl]benzoic acid, methyl ester |
| carbetamide | N-ethyl-2-[[(phenylamino)carbonyl]oxy]propanamide (R)-isomer |
| dalapon | 2,2-dichloropropionic acid |
| diallate | S-(2,3-Dichloroallyl)diisopropylthiocarbamate |
| diuron | 3-(3,4-dichlorophenyl)-1,1-dimethylurea |
| metazachlor | 2-chloro-N-(2,6-dimethylphenyl)-N-(1H-pyrazol-1-yl methyl)acetamide |
| napropamide | 2-(α-Naphthoxy)-N,N-diethyl propionamide |
| nitralin | 4-methylsulfonyl-2,6-dinitro-N,N-dipropylaniline |
| propyzamide | 3,5-dichloro(N-1,1-dimethyl)-2-propynyl)benzamide |
| TCA | trichloroacetic acid |
| 3,6-dichloropicolinic acid | 3,6-Dichloro-2-pyridinecarboxylic acid |
| "Cinch" | exo-1-methyl-4-(1-methylethyl)-2-[(2-methylphenyl)methoxy]-7-oxabicyclo[2.2.1]heptane |

The selective herbicidal properties of the subject compounds were discovered in a number of greenhouse tests conducted as described below.

Test A

Seeds of crabgrass (*Digitaria* spp.), barnyardgrass (*Echinochloa crusgalli*), *Cassia tora*, wild oats (*Avena fatua*), morningglory (*Ipomoea* spp.), cocklebur (*Xanthium pensylvanicum*), sorghum, corn, soybean, sugarbeet, cotton, rice, wheat and purple nutsedge (*Cyperus rotundus*) tubers were planted and treated preemergence with the test chemicals dissolved in a non-phytotoxic solvent. At the same time, cotton having five leaves (including cotyledonary ones), crabgrass, barnyardgrass, and wild oats with two leaves, cassia with three leaves (including cotyledonary ones), morningglory and cocklebur with four leaves (including the cotyledonary ones), sorghum and corn with four leaves, soybean with two primary leaves, rice with three leaves, wheat with one leaf and nutsedge with three-five leaves were sprayed.

Treated plants and controls were maintained in a greenhouse for sixteen days, after which all species were compared to controls and visually rated for response to treatment. The ratings, summarized in Table A, are based on a numerical scale extending from 0 = no injury, to 10 = complete kill. The accompanying descriptive symbols have the following meanings:

C = chlorosis/necrosis;
B = burn;
D = defoliation;
E = emergence inhibition;
G = growth retardation;
H = formative effect;
U = unusual pigmentation;

X = axillary stimulation;
S = albinism; and
6Y = abscised buds or flowers.

Test results are given in Table A.

COMPOUNDS

Compound 1

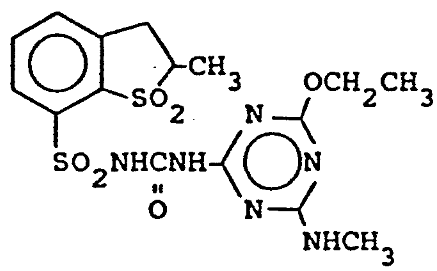

Compound 2

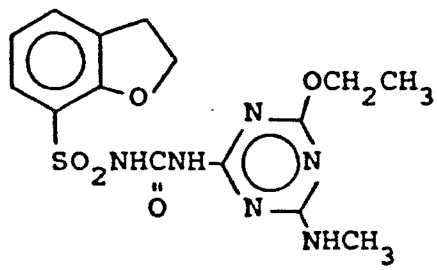

Table A

Compound 1

| Rate g/ha | Postemergence 50 | Preemergence 50 |
|---|---|---|
| Rice | 9C | 10E |
| Wheat | 9G,2C | 9C |
| Corn | 9G,5C | 9G,4C |
| Sorghum | 9G,5C | 9H,2C |
| Soybean | 8H,3C | 8H,3C |
| Sugarbeet | 9C | 9G,4C |
| Cotton | 8G,4C | 2G |
| Nutsedge | 3G | 0 |
| Crabgrass | 8G,3C | 6G,2C |
| Barnyardgrass | 9C | 9H |
| Wild Oats | 9G,5C | 9G,4C |
| Morningglory | 9G,5C | 9G |
| Cocklebur | 9G,4C | 8H |
| Sicklepod | 9G,5C | 7G |

Table A (continued)

Compound 2

| Rate g/ha | Postemergence 50 | Preemergence 50 |
|---|---|---|
| Rice | 9C | 9H,3C |
| Wheat | 9G,2C | 7H,2C |
| Corn | 9G,3C | 9G |
| Sorghum | 9H,4C | 9H,3C |
| Soybean | 8H,3C | 4H,2C |
| Sugarbeet | 8H,4C | 7G,2C |
| Cotton | 9H,4C | 6G |
| Nutsedge | 3G | 0 |
| Crabgrass | 4G | 0 |
| Barnyardgrass | 8H,4C | 5G,2C |
| Wild Oats | 9G,5C | 7G,2C |
| Morningglory | 5H,3C | 7H |
| Cocklebur | 3G | 5G |
| Sicklepod | 8G,4C | 5H,3C |

Test B

Postemergence

Two round pans (25 cm diameter by 12.5 cm deep) were filled with Sassafras sandy loam soil. One pan was planted with blackgrass (*Alopecurus myosuroides*), sugarbeets, nutsedge (*Cyperus rotundus*) tubers, rape (*Brassica napus*), crabgrass (*Digitaria sanguinalis*), sicklepod (*Cassia obtusifolia*), teaweed (*Sida spinosa*), jimsonweed (*Datura stramonium*), velvetleaf (*Abutilon theophrasti*), and giant foxtail (*Setaria faberii*). The other pan was planted with wheat, cotton, rice, corn, soybean, wild oats (*Avena fatua*), cocklebur (*Xanthium pensylvanicum*), morningglory (*Ipomoea hederacea*), johnsongrass (*Sorghum halepense*) and barnyardgrass (*Echinochloa crusgalli*). The plants were grown for approximately fourteen days, then sprayed postemergence with the chemicals dissolved in a non-phytotoxic solvent.

Preemergence

Two round pans (25 cm diameter by 12.5 cm deep) were filled with Sassafras sandy loam soil. One pan was planted with blackgrass, sugarbeets, nutsedge, rape, crabgrass, sicklepod, teaweed, jimsonweed, velvetleaf, and giant foxtail. The other pan was planted with wheat, cotton, rice, corn, soybeans, wild oats, cocklebur, morningglory, johnsongrass, and barnyardgrass. The two pans were sprayed preemergence with the chemicals dissolved in a non-phytotoxic solvent.

Treated plants and controls were maintained in the greenhouse for 28 days, then all treated plants were visually rated and compared with the controls. The response ratings are based on a scale of 0 to 100 where 0 = no effect, 10 = minimal effect and 100 = complete control. The results are given in Table B.

Table B

Compound 1

| | Postemergence | | | | Preemergence | | | |
|---|---|---|---|---|---|---|---|---|
| Rate g/ha | 250 | 62 | 16 | 4 | 250 | 62 | 16 | 4 |
| Corn | 100 | 100 | 100 | 90 | 80 | 20 | 0 | 0 |
| Wheat | 100 | 100 | 100 | 40 | 60 | 40 | 20 | 0 |
| Rice | 100 | 100 | 100 | 80 | 100 | 100 | 90 | 50 |
| Soybean | 90 | 60 | 20 | 0 | 20 | 0 | 0 | 0 |
| Cotton | 0 | 0 | 0 | 0 | 0 | 0 | 0 | 0 |
| Sugarbeet | 100 | 100 | 100 | 50 | 50 | 30 | 0 | 0 |
| Rapeseed | 20 | 0 | 0 | 0 | 0 | 0 | 0 | 0 |
| Crabgrass | 90 | 70 | 30 | 0 | 60 | 30 | 0 | 0 |
| Johnsongrass | 100 | 100 | 80 | 30 | 100 | 90 | 60 | 20 |
| Blackgrass | 100 | 100 | 100 | 80 | 100 | 100 | 90 | 50 |
| Barnyardgrass | 100 | 100 | 90 | 30 | 90 | 80 | 40 | 0 |
| Nutsedge | 60 | 20 | 0 | 0 | 80 | 40 | 0 | 0 |
| Giant Foxtail | 100 | 100 | 20 | 20 | 90 | 70 | 40 | 0 |
| Wild Oats | 100 | 100 | 70 | 20 | 90 | 80 | 40 | 0 |
| Cocklebur | 40 | 30 | 0 | 0 | 20 | 0 | 0 | 0 |
| Morningglory | 70 | 90 | 30 | 0 | 30 | 0 | 0 | 0 |
| Teaweed | 80 | 60 | 40 | 30 | 20 | 0 | 0 | 0 |
| Sicklepod | 80 | 70 | 30 | 0 | 20 | 0 | 0 | 0 |
| Jimsonweed | 100 | 100 | 70 | 40 | 80 | 40 | 0 | 0 |
| Velvetleaf | 100 | 100 | 70 | 20 | 60 | 0 | 0 | 0 |

Compound 2

| Rate g/ha | Postemergence 62 | 16 | 4 | Preemergence 250 | 62 | 16 |
|---|---|---|---|---|---|---|
| Corn | 100 | 90 | 80 | 40 | 20 | 0 |
| Wheat | 0 | 0 | 0 | 0 | 0 | 0 |
| Rice | 90 | 60 | 30 | 100 | 90 | 70 |
| Soybean | 70 | 60 | 20 | 20 | 0 | 0 |
| Cotton | 60 | 20 | 0 | 0 | 0 | 0 |
| Sugarbeet | 40 | 20 | 0 | 0 | 0 | 0 |
| Rapeseed | 0 | 0 | 0 | 0 | 0 | 0 |
| Crabgrass | 0 | 0 | 0 | 0 | 0 | 0 |
| Johnsongrass | 90 | 70 | 50 | 95 | 90 | 60 |
| Blackgrass | 80 | 70 | 20 | 80 | 30 | 0 |
| Barnyardgrass | 60 | 20 | 0 | 50 | 30 | 0 |
| Nutsedge | 0 | 0 | 0 | 0 | 0 | 0 |
| Giant Foxtail | 50 | 0 | 0 | 60 | 30 | 0 |
| Wild Oats | 80 | 60 | 0 | 0 | 0 | 0 |
| Cocklebur | 0 | 0 | 0 | 0 | 0 | 0 |
| Morningglory | 30 | 0 | 0 | 0 | 0 | 0 |
| Teaweed | 0 | 0 | 0 | 0 | 0 | 0 |
| Sicklepod |  | 80 | 0 | 0 | 0 | 0 |
| Jimsonweed | 60 | 20 | 0 | 0 | 0 | 0 |
| Velvetleaf | 90 | 80 | 60 | 0 | 0 | 0 |

Test C

Two plastic windowsill trays were filled with planting medium. One was planted with seeds of speedwell (Veronica persica), Galium aparine, chickweed (Stellaria media), redroot pigweed (Amaranthus retroflexus), wild mustard (Brassica kaber), wild raddish (Raphanus raphanistrum), wild buckwheat (Polygonum convolvulus), common lambsquarters (Chenopodium album), stinkweed (Thlaspi arvense), smartweed (Polygonum spp.), Matricaria inodora and rape (Brassica napus and B. campestris). The other tray was planted with seeds of Italian ryegrass (Lolium multiflorum), green foxtail (Setaria viridis), wild oats (Avena fatua), blackgrass (Alopecurus myosuroides), wheat (Triticum aestivum-vars. 'Nacozari', 'Centurk' and 'Park') and barley (Hordeum vulgare-vars. 'Klages' and 'Morex'). In one test the crops rape, corn, sorghum, soybean, sugarbeet, cotton, alfalfa, wheat ('Nacozari') and barley ('Morex') and the weeds shattercane (Sorghum bicolor), wild proso millet (Panicum milliacenm), johnsongrass (Sorghum halepense), wild oats (Avena fatua), green foxtail (Setaria viridis), giant foxtail (S. faberi), and guineagrass (Panicum maximum) were used.

The trays were treated prior to weed and crop emergence (preemergence) and when crops and weeds had grown to 2 to 3-leaf stage (postemergence) with compounds formulated in a non-phytotoxic solvent. The degree of crop injury and weed control was visually rated 2 to 4 weeks after compound application. The plant response ratings were either based on the scale of 0 to 100 where 0 = no effect, 10 = minimal injury and 100 = complete control or on a scale of 0 to 10 where 0 = no effect and 10 = complete control. The type of response is represented by letters where G = growth retardation. The results are given in Table C.

Table C

Compound 1

| Rate g/ha | Postemergence 125 | 64 | 32 | 16 |
|---|---|---|---|---|
| Canada rape | 0 | 0 | 0 | 0 |
| Wheat | | | | |
| Nacozari | 100 | 100 | 80 | 90 |
| Centurk | 100 | 100 | 80 | 80 |
| Park | 100 | 100 | 90 | 100 |
| Barley | | | | |
| Klages | 100 | 100 | 80 | 80 |
| Morex | 100 | 100 | 100 | 100 |
| Wild oats | 100 | 90 | 80 | 80 |
| Blackgrass | 90 | 90 | 80 | 70 |
| Ryegrass | 100 | 100 | 90 | 80 |
| Green foxtail | 100 | 100 | 90 | 80 |
| Speedwell | 0 | 0 | 0 | 0 |
| Chickweed | 0 | 0 | 0 | 0 |
| Pigweed | 100 | 100 | 100 | 100 |
| Wild mustard | 100 | 100 | 100 | 100 |
| Wild raddish | 0 | 0 | 0 | 0 |
| Wild buckwheat | 0 | 0 | 0 | 0 |
| Lambsquarters | 90 | 90 | 80 | 50 |
| Stinkweed | 60 | 50 | 60 | 60 |

Compound 1

| Rate g/ha | Postemergence 125 | 64 | 16 |
|---|---|---|---|
| Westar rape | 2G | 0 | 0 |
| Corn | 10G | 10G | 10G |
| Sorghum | 10G | 10G | 10G |
| Sugarbeet | 4G | 3G | 3G |
| Proso millet | 10G | 6G | 7G |
| Shattercane | 10G | 10G | 10G |
| Johnsongrass | 10G | 10G | 9G |
| Wild oats | 10G | 9G | 9G |
| Annual rye | 10G | 9G | 9G |
| Cotton | 2G | 0 | 0 |
| Alfalfa | 3G | 0 | 0 |
| Green foxtail | 10G | 8G | 7G |
| Giant foxtail | 6G | 4G | 4G |
| Soybean | 8G | 6G | 6G |
| Wheat ('Nacozari') | 10G | 9G | 9G |
| Barley ('Morex') | 10G | 9G | 8G |
| Guineagrass | 3G | 0 | 0 |

Compound 2

Postemergence

| Rate g/ha | 125 | 64 | 32 | 16 |
|---|---|---|---|---|
| Canada rape | 0 | 0 | 0 | 0 |
| Wheat | | | | |
| Nacozari | 70 | 70 | 20 | 0 |
| Centurk | 30 | 30 | 0 | 0 |
| Park | 80 | 70 | 30 | 0 |
| Barley | | | | |
| Klages | 0 | 0 | 0 | 0 |
| Morex | 80 | 60 | 20 | 20 |
| Wild oats | | | | |
| Blackgrass | 90 | 60 | 20 | 0 |
| Ryegrass | 80 | 70 | 60 | 0 |
| Green foxtail | 90 | 80 | 70 | 50 |
| Speedwell | 0 | 0 | 0 | 0 |
| Chickweed | 20 | 0 | 0 | 0 |
| Pigweed | 100 | 100 | 90 | 70 |
| Wild mustard | 100 | 100 | 100 | 100 |
| Wild raddish | 20 | 0 | 0 | 0 |
| Wild buckwheat | 80 | 80 | 80 | 60 |
| Lambsquarters | 20 | 0 | 0 | 0 |
| Stinkweed | 100 | 60 | 40 | 0 |

Test D

The object of this test was to evaluate compounds which had shown utility for postemergence weed control in rape in combination with other herbicides. Rape, wild mustard, stinkweed, grass weeds and volunteer cereals were sprayed postemergence with the compounds in a non-phytotoxic solvent. Plants were visually rated 21 days-after-treatment (DAT). The rating system was the same as used in Test C. The results are shown in Table D.

Table D1

Plant Injury Rating 21 DAT

Run I Rape 3-leaf: grasses 2 to 3-leaf stage

| | Compound 1 | | | Compound A | |
|---|---|---|---|---|---|
| Rate g/ha | 64 | 32 | 16 | 32 | 16 |
| POSTEMERGENCE | | | | | |
| Westar rape | 0 | 0 | 0 | 0 | 0 |
| Blackgrass | 10G | 10G | 9G | 6G | 5G |
| Annual Rye | 10G | 9G | 9G | 7G | 7G |
| Wild Oats | 10G | 9G | 8G | 4G | 2G |
| Green Foxtail | 10G | 9G | 9G | 4G | 4G |
| Wheat | | | | | |
| Nacozari | 10G | 10G | 10G | 2G | 0 |
| Park | 10G | 10G | 10G | 1G | 0 |
| Centurk | 10G | 10G | 10G | 0 | 0 |
| Barley | | | | | |
| Klages | 9G | 9G | 9G | 0 | 0 |
| Morex | 9G | 9G | 9G | 0 | 0 |
| Stinkweed | 10G | 10G | 10G | 10G | 10G |

| | Compound A + Compound 1 | | | |
|---|---|---|---|---|
| Rate g/ha | 32+32 | 32+16 | 16+32 | 16+16 |
| POSTEMERGENCE | | | | |
| Westar rape | 1G | 0 | 0 | 0 |
| Blackgrass | 10G | 10G | 10G | 9G |
| Annual Rye | 10G | 9G | 9G | 9G |
| Wild Oats | 10G | 10G | 10G | 8G |
| Green Foxtail | 9G | 8G | 10G | 7G |
| Wheat | | | | |
| Nacozari | 10G | 9G | 9G | 8G |
| Park | 10G | 9G | 10G | 10G |
| Centurk | 10G | 9G | 10G | 10G |
| Barley | | | | |
| Klages | 10G | 8G | 9G | 7G |
| Morex | 10G | 8G | 9G | 7G |
| Stinkweed | 10G | 10G | 10G | 10G |

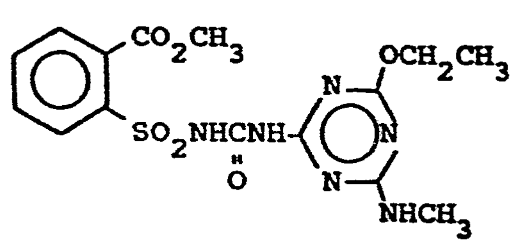

Compound A

Table D2

**Run II Rape 4 to 5-leaf; grasses 4 to 5-leaf stage**

| | Compound 1 | | | Compound A | |
|---|---|---|---|---|---|
| Rate g/ha | 64 | 32 | 16 | 32 | 16 |
| POSTEMERGENCE | | | | | |
| Westar rape | 0 | 0 | 0 | 0 | 0 |
| Blackgrass | 9G | 8G | 8G | 4G | 3G |
| Annual Rye | 8G | 8G | 8G | 5G | 3G |
| Wild Oats | 8G | 8G | 9G | 2G | 3G |
| Green Foxtail | 8G | 8G | 7G | 4G | 4G |
| Wheat | | | | | |
| Nacozari | 9G | 8G | 8G | 0 | 0 |
| Park | 10G | 8G | 8G | 0 | 0 |
| Centurk | 10G | 8G | 8G | 0 | 0 |
| Barley | | | | | |
| Klages | 8G | 8G | 7G | 0 | 0 |
| Morex | 8G | 7G | 7G | 0 | 0 |

| | Compound A | + | Compound 1 | |
|---|---|---|---|---|
| Rate g/ha | 32+32 | 32+16 | 16+32 | 16+16 |
| POSTEMERGENCE | | | | |
| Westar rape | 1G | 0 | 0 | 0 |
| Blackgrass | 9G | 9G | 9G | 8G |
| Annual Rye | 9G | 9G | 8G | 8G |
| Wild Oats | 10G | 9G | 10G | 8G |
| Green Foxtail | 8G | 7G | 8G | 7G |
| Wheat | | | | |
| Nacozari | 10G | 9G | 10G | 7G |
| Park | 10G | 9G | 10G | 7G |
| Centurk | 10G | 9G | 10G | 7G |
| Barley | | | | |
| Klages | 8G | 8G | 9G | 6G |
| Morex | 8G | 9G | 8G | 6G |

Test E

Plastic pots were filled with planting medium. The individual pots were seeded with red clover, alfalfa, sunflower, lambsquarters (Chenopodium spp.), stinkweed (Thlaspi arvense), wild mustard (Brassica kaber), Brown mustard, Oriental mustard, Yellow mustard, sugarbeet, wild radish (Raphanus raphanistrum), rape ('Jet Neuf', 'Altex', 'Tobin' and 'Westar') and flax.

The pots were treated postemergence when the plants had grown to 2 to 5-leaf stage with the compound formulated in a non-phytotoxic carrier made up of acetone-humectant-water-surfactant (22:1:1:0.05 v/v). The degree of crop injury and weed control was visually rated 15 days-after-treatment (DAT). The plant response ratings were based on the scale of 0 to 10 where 0 = no effect, 10 = complete control. The type of response is represented by letters where G = growth retardation. The results are shown in Tables E1 and E2.

Table E1

Plant Injury Rating 15 DAT

| | Compound 1 | | | | |
|---|---|---|---|---|---|
| Rate g/ha | 64 | 32 | 16 | 8 | Check 0 |
| Red Clover | 3G | 2G | 0 | 0 | 0 |
| Alfalfa | 2G | 1G | 0 | 0 | 0 |
| Sunflower | 6G | 2G | 0 | 0 | 0 |
| Lambsquarters | 4G | 3G | 2G | 2G | 0 |

Table E2

Plant Injury Rating 15 DAT

Compound 1

| Rate g/ha | 64 | 32 | 16 | 8 | Check 0 |
|---|---|---|---|---|---|
| Stinkweed | 8G | 8G | 7G | 6G | 0 |
| Mustard | | | | | |
| Wild | 8G | 8G | 8G | 7G | 0 |
| Brown | 0 | 0 | 0 | 0 | 0 |
| Oriental | 0 | 0 | 0 | 0 | 0 |
| Yellow | 0 | 0 | 0 | 0 | 0 |
| Sugarbeet | 7G | 6G | 2G | 0 | 0 |
| Wild Radish | 6G | 5G | 5G | 5G | 0 |
| Rape | | | | | |
| Jet Neuf | 0 | 0 | 0 | 0 | 0 |
| Altex | 0 | 0 | 0 | 0 | 0 |
| Tobin | 0 | 0 | 0 | 0 | 0 |
| Westar | 0 | 0 | 0 | 0 | 0 |
| Flax | 0 | 0 | 0 | 0 | 0 |

Test F

Individual plastic pots filled with planting medium were seeded with lettuce, carrot, kale, kohlrabi, mustard green, turnip, broccoli, brussel sprouts, cauliflower and cabbage. Other pots were planted with russian thistle (Salsola spp.), blackgrass (Alopecurus myosuroides), giant foxtail (Setaria faberi), barnyardgrass (Echinochloa crusgalli) and Galium aparine.

Plants were treated postemergence with the compound formulated with a non-phytotoxic carrier. Plants were visually rated 15 and 18 days-after-treatment (DAT). The response ratings were based on the scale of 0 to 100 where 0 = no effect, 10 = minimal injury and 100 = complete control. The results are shown in Tables F1, F2 and F3.

Table F1

The plants were treated at the 3-leaf stage of growth.

Plant Injury Rating 15 DAT

Compound 1

| Rate g/ha | 64 | 32 | 16 | Check 0 |
|---|---|---|---|---|
| Lettuce | 70 | 40 | 10 | 0 |
| Carrot | 90 | 80 | 70 | 0 |
| Kale | 0 | 0 | 0 | 0 |
| Kohlrabi | 0 | 0 | 0 | 0 |
| Mustard Green | 0 | 0 | 0 | 0 |
| Turnip | 0 | 0 | 0 | 0 |
| Broccoli | 10 | 0 | 0 | 0 |
| Brussel Sprouts | 20 | 10 | 0 | 0 |
| Cauliflower | 20 | 20 | 0 | 0 |
| Cabbage | 10 | 0 | 0 | 0 |

Table F2

The plants were treated at the 4 to 5-leaf stage of growth.

Plant Injury Rating 15 DAT

Compound 1

| Rate g/ha | 64 | 32 | 16 | Check 0 |
|---|---|---|---|---|
| Lettuce | 70 | 30 | 0 | 0 |
| Carrot | 90 | 80 | 0 | 0 |
| Kale | 0 | 0 | 0 | 0 |
| Kohlrabi | 0 | 0 | 0 | 0 |
| Mustard Green | 0 | 0 | 0 | 0 |
| Turnip | 0 | 0 | 0 | 0 |
| Broccoli | 0 | 0 | 0 | 0 |
| Brussel Sprouts | 0 | 0 | 0 | 0 |
| Cauliflower | 0 | 0 | 0 | 0 |
| Cabbage | 0 | 0 | 0 | 0 |

Table F3

Plant Injury Rating 18 DAT

Compound 1

| Rate g/ha | 125 | 64 | 32 | 16 | Check 0 |
|---|---|---|---|---|---|
| Russian Thistle | 90 | 90 | 70 | 70 | 0 |
| Blackgrass | 100 | 100 | 80 | 70 | 0 |
| Giant Foxtail | 90 | 90 | 80 | 70 | 0 |
| Barnyardgrass | 100 | 100 | 100 | 80 | 0 |
| Galium | 30 | 20 | 0 | 0 | 0 |

Test G

The object of this test was to evaluate Compound 1 for postemergence safety to crops and weed control. Cotton, sunflower, wild oats (Avena fatua), flax, green foxtail (Setaria viridis), wheat and safflower were sprayed postemergence with the compound in a non-phytotoxic solvent. Plants were visually rated 20 days-after-treatment (DAT). The rating system was the same as used in Test F. The results are shown in Table G.

Table G

Plant Injury Rating 20 DAT

Compound 1

| Rate g/ha | 64 | 32 | 16 | 8 | Check 0 |
|---|---|---|---|---|---|
| Cotton | 20 | 10 | 0 | 0 | 0 |
| Sunflower | 90 | 70 | 60 | 10 | 0 |
| Wild Oats | 100 | 100 | 100 | 80 | 0 |
| Flax | 10 | 0 | 0 | 0 | 0 |
| Green Foxtail | 100 | 80 | 80 | 70 | 0 |
| Wheat | 100 | 100 | 100 | 80 | 0 |
| Safflower | 30 | 20 | 20 | 0 | 0 |

For the contracting states:
BE CH DE FR GB GR
IT LI LU NL SE

BA-8680-A

Claims:

1. A compound selected from

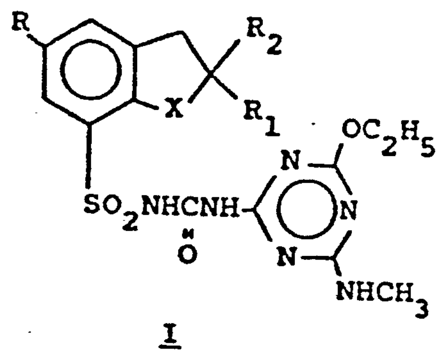

I

wherein

R is H, Cl, F, $CH_3$ or $OCH_3$;
$R_1$ is H or $CH_3$;
$R_2$ is H, $CH_3$ or $C_2H_5$; and
X is O, S or $SO_2$;

provided that when $R_1$ and $R_2$ are simultaneously H, then X is O, and agriculturally suitable salts thereof.

2. Compounds of Claim 1 where R is H.

3. Compounds of Claim 2 where $R_1$ is H; and $R_2$ is $CH_3$.

4. The compound of Claim 1, N-[[4-ethoxy-6-(methylamino)-1,3,5-triazin-2-yl]aminocarbonyl]-2,3-dihydro-2-methylbenzo[b]thiophene-7-sulfonamide, 1,1-dioxide, and agriculturally suitable salts thereof.

5. A composition suitable for controlling the growth of undesired vegetation which comprises an effective amount of a compound of any of Claims 1 to 4 and at least one of the following: surfactant, solid or liquid diluent.

6. A method for controlling the growth of undesired vegetation which comprises applying to the locus to be protected an effective amount of a compound of any of claims 1 to 4.

7. A method for controlling the growth of undesired vegetation in a crop of the genus Brassica which comprises applying to the locus to be protected an effective amount of a compound of any of claims 1 to 3.

8. The method of claim 7 wherein the compound of claim 4 is applied.

9. The method of claim 7 or 8 wherein said crop is oilseed rape.

10. The method of claim 7 or 8 wherein said crop is cultivated mustard.

11. A mixture comprising a herbicidally effective amount of 2-[[(4-ethoxy-6-methylamino-1,3,5-triazin-2-yl)aminocarbonyl]aminosulfonyl]-benzoic acid, methyl ester or an agriculturally suitable salt thereof and a herbicidally effective amount of N-[[4-ethoxy-6-(methylamino)-1,3,5-triazin-2-yl]aminocarbonyl]-2,3-dihydro-2-methylbenzo[b]-thiophene-7-sulfonamide, 1,1-dioxide, or an agriculturally suitable salt thereof.

12. A composition suitable for controlling the growth of undesired vegetation which comprises an effective amount of the mixture of Claim 11 and at least one of the following: surfactant, solid or liquid diluent.

13. A method for controlling the growth of undesired vegetation which comprises applying to the locus to be protected an effective amount of the mixture of Claim 11.

14. A method for controlling the growth of undesired vegetation in a crop of the genus Brassica which comprises applying to the locus to be protected an effective amount of the mixture of Claim 11.

15. The method of Claim 14 wherein said crop is oilseed rape.

16. The method of Claim 14 wherein said crop is cultivated mustard.

17. A method for regulating the growth of plants which comprises applying to the locus of such plants an effective but substantially non-phytotoxic amount of a plant growth regulant selected from compounds of any of claims 1 to 4.

18. A process for the preparation of a compound of claim 1 which comprises:

(a) reacting a sulfonylisocyanate of Formula

$$JSO_2NCO \qquad (II)$$

with a heterocyclic amine of Formula

$$NH_2\text{-}A \qquad (III);$$ or

(b) reacting a sulfonylcarbamate of Formula

$$JSO_2NHCO_2Ph \qquad (IV)$$

with said amine of Formula (III); or

(c) reacting a sulfonamide of Formula

$$JSO_2NH_2 \qquad (V)$$

with the appropriate carbamate heterocycle of Formula

$$C_6H_5OC(=O)NH\text{-}A \qquad (VI);$$

wherein J is 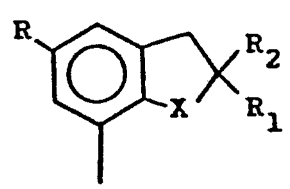

where R, $R_1$, $R_2$ and X are as defined in claim 1,

and A is 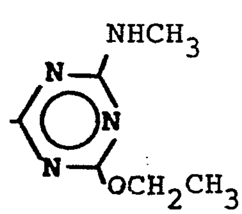

For the contracting states: AT ES

Claims:

1. A process for the preparation of a compound selected from

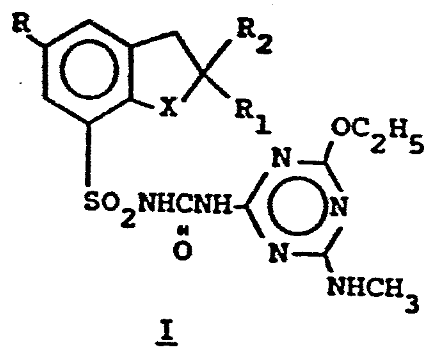

I

wherein

R is H, Cl, F, $CH_3$ or $OCH_3$;
$R_1$ is H or $CH_3$;
$R_2$ is H, $CH_3$ or $C_2H_5$; and
X is O, S or $SO_2$;

provided that when $R_1$ and $R_2$ are simultaneously H, then X is O, and agriculturally suitable salts thereof, which comprises:

(a) reacting a sulfonylisocyanate of Formula

$$JSO_2NCO \qquad (II)$$

with a heterocyclic amine of Formula

$$NH_2\text{-}A \qquad (III)$$; or

(b) reacting a sulfonylcarbamate of Formula

$$JSO_2NHCO_2Ph \qquad (IV)$$

with said amine of Formula (III); or

(c) reacting a sulfonamide of Formula

$$JSO_2NH_2 \qquad (V)$$

with the appropriate carbamate heterocycle of Formula

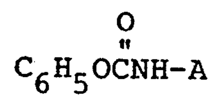 (VI);

wherein J is 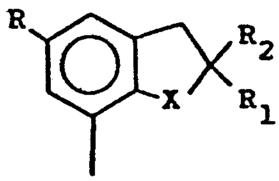

where R, $R_1$, $R_2$ and X are as defined above,

and A is 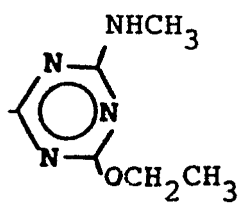.

2. A process of Claim 1 where R is H.

3. A process of Claim 2 where $R_1$ is H; and $R_2$ is $CH_3$.

4. The process of Claim 1 wherein the product is N-[[4-ethoxy-6-(methylamino)-1,3,5-triazin-2-yl]-aminocarbonyl]-2,3-dihydro-2-methylbenzo[b]thiophene-7-sulfonamide, 1,1-dioxide, or an agriculturally suitable salt thereof.

5. A composition suitable for controlling the growth of undesired vegetation which comprises an effective amount of a compound of Formula (I) as defined in any of Claims 1 to 4 and at least one of the following: surfactant, solid or liquid diluent.

6. A method for controlling the growth of undesired vegetation which comprises applying to the locus to be protected an effective amount of a compound of Formula (I) as defined in any of claims 1 to 4.

7. A method for controlling the growth of undesired vegetation in a crop of the genus Brassica which comprises applying to the locus to be protected an effective amount of a compound of Formula (I) as defined in any of claims 1 to 3.

8. The method of claim 7 wherein N-[[4-ethoxy-6-(methylamino)-1,3,5-triazin-2-yl]aminocarbonyl]-2,3-dihydro-2-methylbenzo[b]thiophene-7-sulfonamide, 1,1-dioxide, or an agriculturally suitable salt thereof is applied.

9. The method of claim 7 or 8 wherein said crop is oilseed rape.

10. The method of claim 7 or 8 wherein said crop is cultivated mustard.

11. A mixture comprising a herbicidally effective amount of 2-[[(4-ethoxy-6-methylamino-1,3,5-triazin-2-yl)aminocarbonyl]aminosulfonyl]-benzoic acid, methyl ester or an agriculturally suitable salt thereof and a herbicidally effective amount of N-[[4-ethoxy-6-(methylamino)-1,3,5-triazin-2-yl]aminocarbonyl]-2,3-dihydro-2-methylbenzo[b]-thiophene-7-sulfonamide, 1,1-dioxide, or an agriculturally suitable salt thereof.

12. A composition suitable for controlling the growth of undesired vegetation which comprises an effective amount of the mixture of Claim 11 and at least one of the following: surfactant, solid or liquid diluent.

13. A method for controlling the growth of undesired vegetation which comprises applying to the locus to be protected an effective amount of the mixture of Claim 11.

14. A method for controlling the growth of undesired vegetation in a crop of the genus Brassica which comprises applying to the locus to be protected an effective amount of the mixture of Claim 11.

15. The method of Claim 14 wherein said crop is oilseed rape.

16. The method of Claim 14 wherein said crop is cultivated mustard.

17. A method for regulating the growth of plants which comprises applying to the locus of such plants an effective but substantially non-phytotoxic amount of a plant growth regulant selected from compounds of Formula (I) as defined in any of claims 1 to 4.